# EUROPEAN PATENT SPECIFICATION

(11) **EP 3 704 228 B1**
(45) Date of publication and mention of the grant of the patent: **16.08.2023**
(21) Application number: 18796906.8
(22) Date of filing: 31.10.2018
(51) Int. Cl.: C12N 5/077, A61K 35/34, C12N 15/113

(54) **SKELETAL MUSCLE DIFFERENTIATION OF MESODERMAL IPSC DERIVED PROGENITORS**
SKELETTMUSKELDIFFERENZIERUNG VON VORLÄUFERN AUS MESODERMALEN IPSC
DIFFÉRENCIATION DE MUSCLE SQUELETTIQUE DE PROGÉNITEURS DÉRIVÉS DE CSPI MÉSODERMIQUE

(30) Priority: 31.10.2017 GB 201717939
(43) Date of publication of application: 09.09.2020
(73) Proprietor: Katholieke Universiteit Leuven, 3000 Leuven (BE)
(72) Inventor: GIACOMAZZI, Giorgia, 3000 Leuven (BE); POZZO, Enrico, 3000 Leuven (BE); SAMPAOLESI, Maurilio, 3000 Leuven (BE)
(74) Representative: Arnold & Siedsma
(86) International application number: PCT/EP2018/079826
(87) International publication number: WO 2019/086515

(56) References cited:
- MATTIA QUATTROCELLI: "Mesodermal iPSC-derived progenitor cells functionally regenerate cardiac and skeletal muscle.", THE JOURNAL OF CLINICAL INVESTIGATION, vol. 12, 1 December 2005 (2005-12-01), pages 4463-4482, XP055540951, DOI: https://www.ncbi.nlm.nih.gov/pmc/articles/ PMC4665797/pdf/JCI82735.pdf cited in the application
- JIWON SHIM ET AL: "The expression and functional roles of microRNAs in stem cell differentiation", BMB REPORTS, vol. 49, no. 1, 31 January 2016 (2016-01-31), pages 3-10, XP055540957, KR ISSN: 1976-6696, DOI: 10.5483/BMBRep.2016.49.1.217
- ALBERTO IZARRA ET AL: "miRNA-1 and miRNA-133a are involved in early commitment of pluripotent stem cells and demonstrate antagonistic roles in the regulation of cardiac differentiation : miRNA-1 and miRNA-133a have antagonistic roles in cardiac differentiation", JOURNAL OF TISSUE ENGINEERING AND REGENERATIVE MEDICINE, vol. 11, no. 3, 10 December 2014 (2014-12-10), pages 787-799, XP055540963, US ISSN: 1932-6254, DOI: 10.1002/term.1977
- GIORGIA GIACOMAZZI ET AL: "MicroRNAs promote skeletal muscle differentiation of mesodermal iPSC-derived progenitors", NATURE COMMUNICATIONS, vol. 8, no. 1, 1 November 2017 (2017-11-01), XP055540965, DOI: 10.1038/s41467-017-01359-w

## Description

### Field of the invention

The present invention relates to the use of induced pluripotent stem cells for muscle repair and methods the improve the myogenic potential of such induced stem cells.

### Background of the invention

Stem cells hold potential for understanding the regeneration mechanisms with possible applications to degenerative disorders [McCullagh & Perlingeiro (2015) Adv Drug Deliv Rev 84, 198-207]. In particular, the recent advancements in the field of induced pluripotent stem cells (iPSCs) are paving the way to multi-tissue differentiation in patient-matched, isogenic settings [Fox et al. (2014) Science 345, 1247391]. This is particularly compelling for multi-tissue degenerative diseases, such as muscular dystrophies (MDs) [Costamagna et al. (2014) Cell Mol Life Sci 71, 615-627; Quattrocelli et al. (2010) Cell Death Differ 17, 1222-1229]. MDs encompass a heterogeneous group of inherited myopathies that affect skeletal muscle but in some subgroups also cardiac muscle [Emery (2002) Lancet 359, 687-695]. At present, no regenerative treatments are available to counteract myofiber and myocyte wastage and functional loss.

The differentiation ability of iPSCs is under the influence of both extrinsic and intrinsic factors [Takahashi & Yamanaka (2015) Development 142, 3274-3285]. Extrinsic factors that direct differentiation include the addition and withdrawal of specific growth factors. Cell-intrinsic factors include the epigenetic factors that influence the propensity of iPSCs towards the intended lineage [Liang & Zhang (2013) Cell Stem Cell 13, 149-159]. Albeit still unclear, the retention of progeny-specific epigenetic imprinting in iPSCs, e.g. patterns of DNA methylation and histone marks, has been often reported and exploited for enhanced differentiation along the parental lineage [Sanchez-Freire et al (2014) J Am Coll Cardiol 64, 436-448**].** However, it is not yet possible to manipulate the aforementioned epigenetic layers in specific loci. Therefore, the research involving the so-called "epigenetic memory" is still mainly descriptive and the main interventional path resides in the choice of the source cells for iPSC generation [Bar-Nur et al. (2011) Cell Stem Cell 9, 17-23]. Thus, the search for epigenetic signatures that can be modulated to specifically alter the differentiation propensity of iPSCs, or their derivatives, is still on.

MicroRNAs (miRs) are well positioned to contribute to epigenetic regulation of differentiation of stem cells [Quattrocelli & Sampaolesi (2015) Adv Drug Deliv Rev 88, 37-52**].** The potential of miR-based orchestration of cell fate is evident along the striated muscle lineages and, recently, miRs have been described as part of the epigenetic signature retained after cell reprogramming [Vitaloni et al. (2014) J Biol Chem 289, 2084-2098]. MiRs are of particular interest because they and their anti-miRs are small and readily deliverable to manipulate differentiation potential. Better definition of transcriptional and miR profiles will assist in the goal of designing cocktails for skewing the differentiation propensity.

Recently, a novel pool of mesodermal, iPSC-derived progenitors (MiPs) for striated muscle regeneration was described [Quattrocelli et al. (2015) J Clin Invest 125, 4463-4482; Sandri (2015); J Clin Invest 125, 4331-4333]. MiPs were sorted as CD140a⁺/CD140b⁺/CD44⁺ cells from differentiating iPSCs of murine, canine, and human origins. Importantly, murine MiPs were able to functionally regenerate both cardiac and skeletal muscles in murine models. Furthermore, the propensity of MiPs toward the skeletal muscle lineage appeared augmented when derived from skeletal muscle mesoangioblasts (MAB-MiPs), as compared to isogenic fibroblast-derived MiPs.

Methods to improve the myogenic potential of the fibroblast-derived MiPs as described in Quattrocelli *et al.* 2015 are required. GeoAccession GSE102283 discloses raw sequencing data and reports that RNA sequencing of MiPs and of induced pluripotent stem cells (iPSCs) of origin allows identification of genes differentially regulated between fibroblast and MABs progenies that might be responsible for the different contribution to muscle regeneration. MicroRNA- sequencing of the same cell lines could lead to a set of miRNAs that can drive the myogenic propensity of MiPs in vivo and identification of promyogenic and antimyogenic miRNA cocktails.

The nature of these genes, and how to arrive at the promyogenic and antimyogenic miRNA cocktails, merely based on the raw sequencing data has however not been revealed.

### Summary of the invention

The present invention discloses the identity of different miRNAs that promote or inhibit development of fibroblast derived (iPSCs) into cells with improved myogenic potential.

The present invention relates to in vitro methods to increase the myogenic potential of mesodermal iPSC derived progenitors of fibroblast origin comprising the step of administering compounds which induce pro-myogenic genes or administering compound which inhibit antimyogenic genes, wherein said compounds are:
- optionally one or more of small interfering RNA against OSTN, MYB, LHX2, LTBP4, BMP6 and SMAD 5, and inhibitors for fibroblast associated miR-34c-5p, miR-34c-3p, miR-362, miR-210, and miR-590, and MAP-MiP associated miR-212, miR -132, miR -424, miR -146b, miR -181a or mimics thereof.

The boosted differentiation of MiPs potential toward skeletal muscle correlated with retained signatures of DNA methylation and histone marks from parental progenies [Quattrocelli et al. (2015) J Clin Invest 125, 4463-4482]. The present invention investigates the *in vivo* capacity of human MiPs, mainly focusing attention on their skeletal myogenic commitment. First, the translational potential of human MiPs was assessed in xenograft-permissive dystrophic mice showing evidence of striated muscle regeneration. The present invention shows that the treatment of MiPs with a selected promyogenic miRNA cocktail further improved MiPs contribution to skeletal muscle regeneration.

Muscular dystrophies (MDs) are often characterized by impairment of both skeletal and cardiac muscle. Regenerative strategies for both compartments therefore constitute a therapeutic avenue. Mesodermal iPSC-derived progenitors (MiPs) can regenerate both striated muscle types simultaneously in mice. Importantly, MiP myogenic propensity is influenced by somatic lineage retention. However, it is still unknown whether human MiPs have *in vivo* potential. Furthermore, methods to enhance the intrinsic myogenic properties of MiPs are likely needed given the scope and need to correct large amounts of muscle in the MDs. The present invention documents that human MiPs can successfully engraft into the skeletal muscle and hearts of dystrophic mice. Utilizing non-invasive live imaging and selectively induced apoptosis, the present invention reports evidence of striated muscle regeneration *in vivo* in mice by human MiPs. Finally, combining RNA-seq and miRNA-seq data, miRNA cocktails are defined that promote the myogenic potential of human MiPs.

The invention is further summarized in the following statements:
1. An in vitro method to increase the myogenic potential of progenitor cells from pluripotent stem cells induced into the mesodermal lineage, the method comprising the step of administering to said progenitor cells miR-212, miR -132, miR -424, miR -146b, and miR -181a or mimics thereof, and inhibitors for miR-34c-5p, miR-34c-3p, miR-362, miR-210, and miR-590.
2. The method according to statement 1, wherein the pluripotent cells are embryonic stem cells.
3. The method according to statement 1, wherein the pluripotent cells are iPSCs.
4. The method according to statement 3, wherein the iPSCs are from fibroblast lineage.
5. The method according to statement 3, wherein the iPSCs are from mesoangioblasts, or from any source of adult somatic cells.
6. The method according to any one of statements 1 to 5, wherein the mixture further comprises small interfering RNA against one more selected from the group of OSTN, MYB, LHX2, LTBP4, BMP6 and SMAD5.
7. The method according to any one of statements 1 to 6, wherein the mixture comprises small interfering RNA against OSTN, MYB, LHX2, LTBP4, BMP6 and SMAD5, and inhibitors for miR-34c-5p, miR-34c-3p, miR-362, miR-210, and miR-590, and miRNA or mimics for miR-212, miR -132, miR -424, miR -146b, and miR -181a.
8. A kit comprising miR-212, miR -132, miR -424, miR -146b, and miR -181a or mimics thereof, and, inhibitors for miR-34c-5p, miR-34c-3p, miR-362, miR-210, and miR-590.
9. The kit according to any one of statements 8, wherein the mixture further comprises small interfering RNA against one more selected from the group of OSTN, MYB, LHX2, LTBP4, BMP6 and SMAD5.
10. The kit according to any one of statements 8 or 9, wherein the mixture comprises small interfering RNA against OSTN, MYB, LHX2, LTBP4, BMP6 and SMAD5, and inhibitors for miR-34c-5p, miR-34c-3p, miR-362, miR-210, and miR-590
   and miRNA or mimics for miR-212, miR -132, miR -424, miR -146b, and miR -181a.
11. In vitro use of a kit according to any one of statements 8 to 10 for increasing the myogenic potential of progenitor cells from pluripotent stem cells induced into the mesodermal lineage.

### Detailed description of the invention

### Figure legends

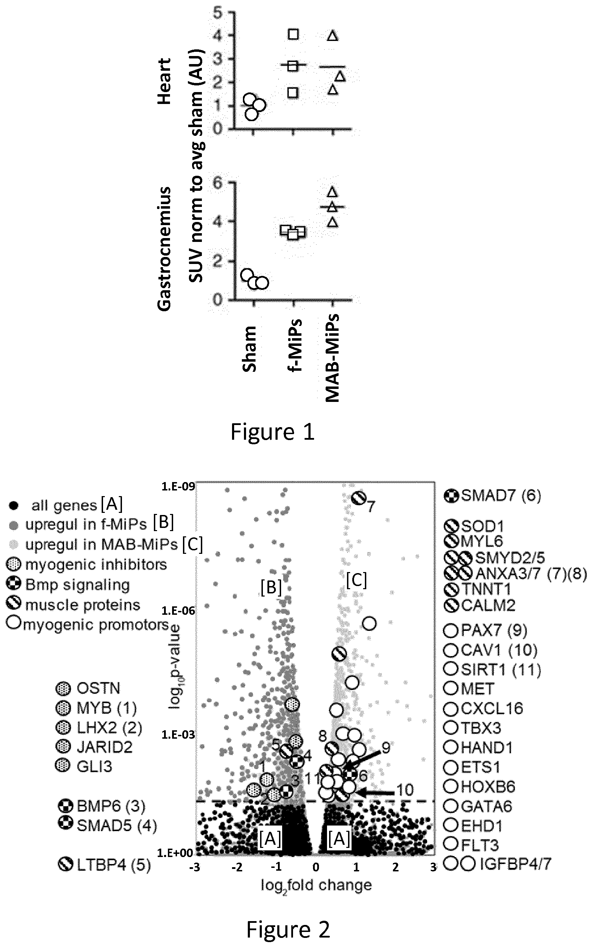

**Figure 1** shows SUV levels in heart and hindlimb regions of PET-scanned mice. *, P<0.05 vs sham; **, P<0.05 vs sham and fibroblast-MiPs; Kruskal Wallis and Mann-Whitney U test; n=3/cohort.
**Figure 2** shows a volcano plot of fold change vs p-value of all DE genes at MiP stage (threshold, P=0.05, dashed line). Left side of the chart, all genes and highlighted candidates enriched in fibroblast-MiPs; right side, genes and candidates enriched in MAB-MiPs.

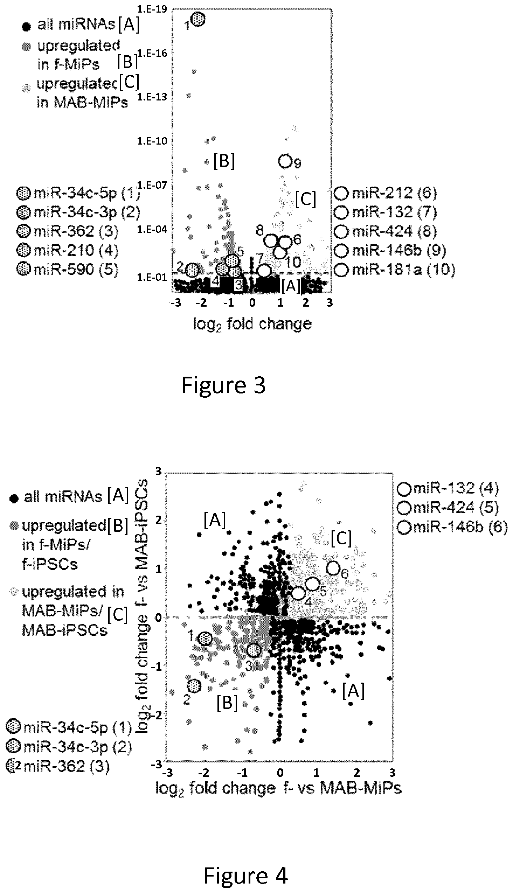

**Figure 3** shows a volcano plot of fold change vs p-value of all DE miRNAs at MiP stage (threshold, P=0.05, dashed line). Left side of the chart, all genes and highlighted candidates (miRNAs predicted to bind 3'UTR of MAB-MiP-upregulated genes) enriched in fibroblast-MiPs; right side, genes and candidates (miRNAs predicted to bind 3'UTR of fibroblast-MiP-upregulated genes) enriched in MAB-MiPs.
**Figure 4** shows a Log2 chart of fold change at MiP stage vs at iPSC stage of signfiicantly DE miRNAs (threshold, P>0.05 at MiP stage). dots in the bottom left quarter depict miRNAs upregulated in fibroblast-iPSCs and fibroblast-MiPs, while dots in the top right quarte depict upregulated miRNAs in MAB-iPSCs and MAB-MiPs. Circled dots represent the candidates, as identified in figure 3.

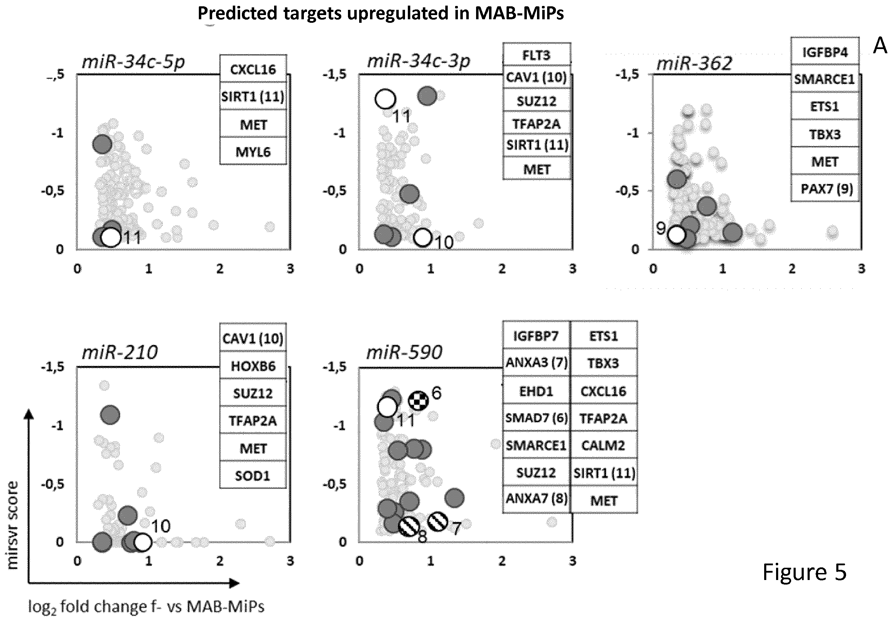

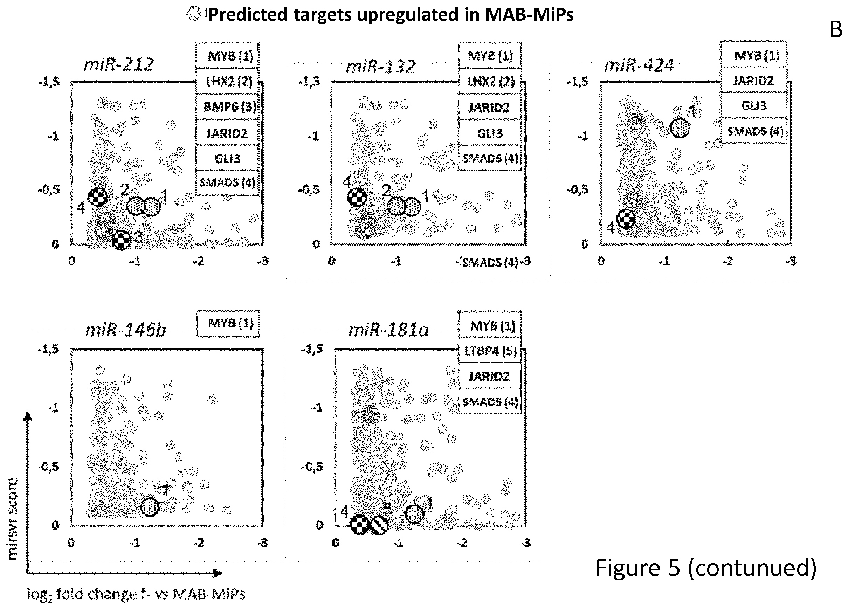

**Figure 5** illustrates the predicted targets of miRNA-seq-based candidates among RNA-seq-based interesting hits mirsvr score vs fold change plots. Charts in (A) depict the predicted targets (interesting hits are highlighted) of fibroblast-MiP-enriched miRNAs among genes upregulated in MAB-MiPs. Charts in (B) depict the predicted targets (interesting hits are highlighted) of MAB-MiP-enriched miRNAs among genes upregulated in fibroblast-MiPs. f-, fibroblast-derived. qPCR-based assay of expression levels of predicted targets of perturbed miRNAs in presence of AMC or PMC. *, P<0.05 vs own ctrl (scramble), Kruskal Wallis and Mann-Whitney U test; n=3 replicates/clone. f-, fibroblast-derived; AMC, anti-myogenic cocktail (miRNA-based), PMC, pro-myogenic cocktail (miRNA-based).

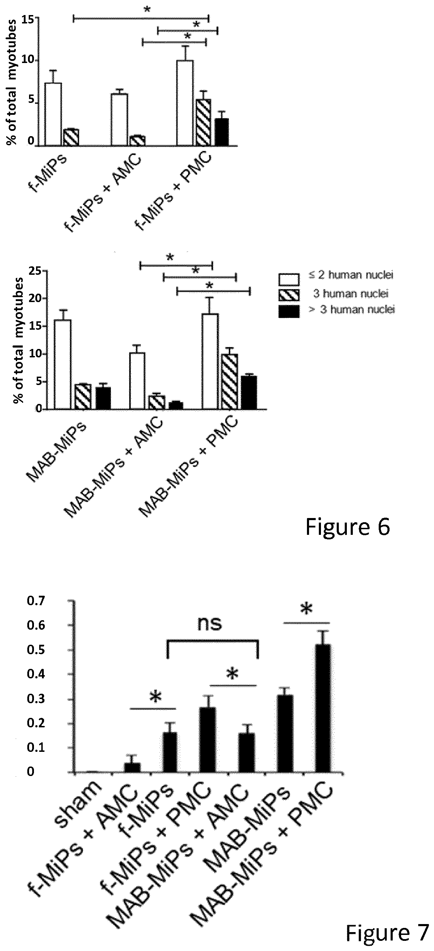

**Figure 6** shows quantification of MiP myogenic propensity of f-MiPS (top) and MAB-MiPs (bottom) in co-culture with C2C12 myoblasts after seven days of differentiation. Myotubes with three or more nuclei were counted as well as human nuclei contributing to chimeric myotubes. Representative fields and quantitation of chimeric myotubes are presented. P<0.05 vs treated and non treated. Kruskal Wallis and Mann-Whitney U test; n=3 replicates per clone. f-, fibroblast-derived; AMC, anti-myogenic cocktail (gene-based), PMC, pro-myogenic cocktail (gene-based), scale bar approximately 100µm
**Figure 7** represents results of hindlimb (gastrocnemius) muscles of dystrophic, immunodeficient mice injected with AMC- or PMC-treated MiPs. The figure shows DYS quantitation by protein analysis. ns: non significant difference; ^{∗}P < 0.05; Kruskal-Wallis test and Mann-Whitney U-test vs own ctrl (scramble oligos), n = 3/cohort. Depicted are average ± st. dev bars. f-, fibroblast-derived, DE, differentially expressed, AMC, anti-myogenic cocktail (miRNA-based), PMC, pro-myogenic cocktail (miRNA-based).

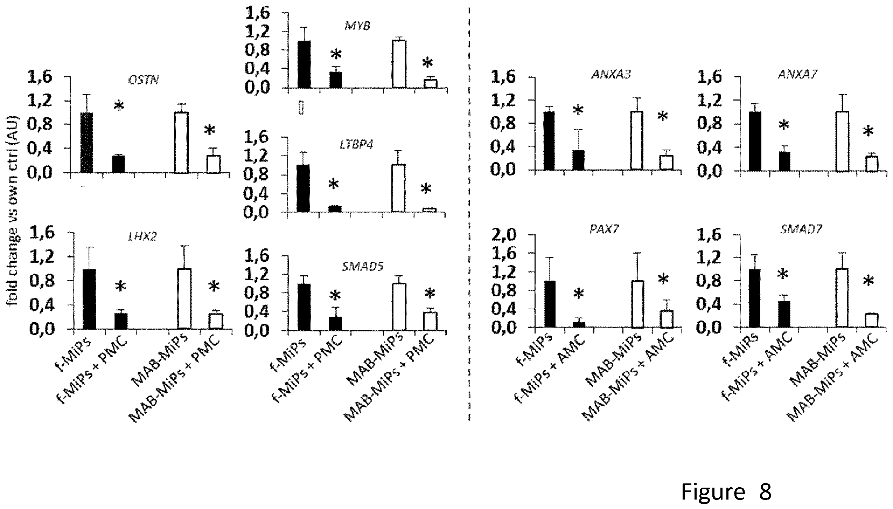

**Figure 8** shows qPCR-based assay of expression levels of predicted targets of perturbed miRNAs in presence of AMC (left part) or (PMC right part). *, P<0.05 vs own ctrl (scramble), Kruskal Wallis and Mann-Whitney U test; n=3replicates/clone. f-, fibroblast-derived; AMC, anti-myogenic cocktail (miRNA-based), PMC, pro-myogenic cocktail (miRNA-based).

The invention relates to methods of differentiation of iPSC-derived cells towards muscle cells.

The invention is typically performed with iPSC-derived progenitor cells that have been differentiated into the mesodermal lineage and have the potential to differentiate into striated muscle cells.

"Induced pluripotent stem cells (iPSC or iPS cells)" are somatic cells that have been reprogrammed, for example, by introducing exogenous genes that confer on the somatic cell a less differentiated phenotype. These iPS cells can then be induced to differentiate into tissue derivatives of the three germ layers. iPS cells have been derived using modifications of an approach originally discovered in 2006 (Yamanaka, et al. (2007) Cell Stem Cell 1, 39-49). For example, in one instance, to create iPS cells, scientists started with skin cells that were then modified by a standard laboratory technique using retroviruses to insert genes into the cellular DNA. In one instance, the inserted genes were Oct4, Sox2, Lif4, and c-myc, known to act together as natural regulators to keep cells in an embryonic stem cell-like state. These cells have been described in the literature. See, for example, Wernig et al. (2008), PNAS, 105, 5856-5861; Jaenisch et al. (2008) Cell 132, 567-582; Hanna et al. (2008) Cell 133, 250-264 (2008); and Brambrink et al. (2008) Cell Stem Cell 2, 151-159 (2008). These references are incorporated by reference for teaching IPSCs and methods for producing them. It is also possible that such cells can be created by specific culture conditions (exposure to specific agents).

It envisaged that the methods are equally applicable to other muscle progenitor cells, such as derived from embryonic stem cells, from satellite cells or other muscle derived cells or from precursor or progenitor cells from other tissues that are differentiated into the mesodermal lineage.

iPSC can be obtained from any somatic tissue. For the purpose of muscle repair in humans the cells can be derived from e.g. muscle biopsies or from other organs such as skin or other organs which can be easily accessed and/or provide sufficient. Tissue biopsies can be cultivated under conditions allowing a certain degree of dedifferentiation or to preserve properties of a mesodermal cell or a muscle progenitor cell.

The cells which are used in the envisaged medical applications, can be allogeneic, but are typically autologous.

The cells are typical human cells for use in the treatment of human diseases.

Cells from other mammals are equally envisaged, e.g. for use in animal muscle disease models such as mice, rats and goats, but also for use in veterinary applications such as horses.

"Progenitor cells" are cells produced during differentiation of a stem cell that have some, but not all, of the characteristics of their terminally-differentiated progeny. Defined progenitor cells, such as "mesodermal progenitor cells" and "cardiac progenitor cells" are committed to a lineage, but not to a specific or terminally differentiated cell type. A progenitor cell can form a progeny cell that is more highly differentiated than the progenitor cell.

"Mesodermal progenitor cells" can be identified using marker known in the art. Mesodermal progenitor cells are obtained from iPSC the cells are characterized in that they all express at least one, at least two, and preferably at least 3 marker genes selected from the group consisting of CD44, CD140a, and CD140b. Said cells all individually express the 2 or more preferably all 3 markers from the group CD44, CD140a, and CD140b. In particular, said CD44- and/or CD140a- and/or CD140b-expressing cells additionally express at least 1, 2, 3, 4, 5, 6, 7, 8, 9, or all 10 markers from the group consisting of Brachyury, MixH, Meoxl, Mespl, Pax3, Pax7, Desmin, Gata4, Tbx5, and Flkl Preferably, said mesodermal progenitor cells express CD44, CD140a, CD140b, and at least 1, 2, 3, 4, 5, 6, 7, 8, 9, or all 10 markers from the group consisting of Brachyury, MixH, Meoxl, Mespl, Pax3, Pax7, Desmin, Gata4, Tbx5, and Flkl Most preferably, said mesodermal progenitor cells express CD44, CD140a, CD140b, Brachyury, MixM, Meoxl, Mespl, Pax3, Pax7, Desmin, Gata4, Tbx5, and Flkl.

"Myogenic potential" refers to capacity of the mesodermal progenitor cells to develop into striated muscle tissue. The potential can be assessed by one or more of molecular markers on the progenitor cells, by structural properties of in vitro cultured cells or by in vivo assays wherein cells are injected in muscles.

The myogenic potential can be assessed by cultivating the progenitor cells together with murine myoblast and determining the amount of myotubes that is formed by differentiated progenitor cells.

The myogenic potential can also be assessed by injection of progenitor cells of a first species (e.g. human) into a muscle of an animal of a second species (e.g. mouse) and determining by species specific antibodies the contribution of cells of the first species in muscle. Other *in vivo* assays comprise injection of cells in a disease model and documenting therapeutic effect of the injected cells.

The present invention identifies throughout the examples section various markers which can be used to identify the osteogenic potential of cells of various origin on a molecular level. OSTN, MYB, LHX2, LTBP4, BMP6 and SMAD5 are examples of anti-myogenic markers. ANXA3/7, SMAD7, CAV1, SIRT1, PAX7, MYL6, ACTA2, TNNT1, HAND1, CXCL16, MET, DLL, CNN2, CNN3, MAST1, GDF15, ATG10 and ATG14 are examples of pro-myogenic markers.

"microRNA", "miRNA" or "miR" refers to short (typically 20-24 nt) non-coding RNAs that are involved in post-transcriptional regulation of gene expression in multicellular organisms by affecting both the stability and translation of messenger RNAs (mRNAs). miRNAs thus act as guide molecules in RNA silencing. They are involved in nearly all developmental and pathological processes in animals. The nomenclature has been standardized in the sequence are publicly available in dedicated databases.

"Inhibitor of miRNA" refers to molecules that interfere with the function of a miRNA (expression or activity) miRNA can originate from a pre-miRNA, an "inhibitor of miRNA" can also be an inhibitor of the corresponding pre-miRNA. The inhibition can be done either at the DNA level (e.g. by interfering with transcription) or at the RNA level (e.g. by interfering with the successive miRNA biogenesis steps, through destabilization of the pri-miRNA so that they are degraded, or typically in case of noncoding RNAs, by interfering with the miRNA itself, e.g. through hybridization). "inhibitor of miRNA" can thus be an "inhibitor of expression" or an "inhibitor of activity".

Inhibiting at the DNA level can for instance be done by inhibiting functional expression of the miR-210 gene itself, but since it requires genomic manipulation it is preferably avoided in the context of cell therapy approaches to prevent neoplastic transformation.

miRNA can also be inhibited at the level of RNA. This can for example be done by the inhibitory RNA technology wherein inhibitors will break down transcribed pri-miR or derivatives along the miRNA biogenesis process. The inhibitory RNA technology or RNA interference (RNAi) is a form of post-transcriptional gene silencing that is used in this application as one of the methods to inhibit or reduce the functional expression. The mediators of sequence-specific messenger RNA degradation are small interfering RNAs (siRNAs) generated by ribonuclease III cleavage from longer dsRNAs. Generally, the length of siRNAs is between 20-25 nucleotides. The siRNAs can be targeted to any stretch of approximately 19 to 25 contiguous nucleotides in miR-210 sequence (the "target sequence"). Techniques for selecting target sequences for siRNA are well known in the art.

Also, antisense oligomers can be used as inhibitors of miRNA expression. An "antisense oligomer" refers to an antisense molecule or anti-gene agent that comprises an oligomer of at least about 10 nucleotides in length. In particular embodiments an antisense oligomer comprises at least 15, 18, 20, 25, 30, 35, 40, or 50 nucleotides. A
Antisense oligomers used to inhibit miR function may consist of DNA, RNA or other, synthetic structures such as phosphorothiates, 2'-0-alkyl ribonucleotide chimeras, locked nucleic acid (LNA) (which will be discussed further), peptide nucleic acid (PNA), morpholinos, gapmer ( chimeric antisense oligonucleotide that contains a central block of deoxynucleotide monomers sufficiently long to induce RNase H cleavage), or antagomirs. (chemically engineered oligonucleotides that can be used to silence endogenous microRNA. An antagomir is a small synthetic RNA that is perfectly complementary to the specific miRNA target with either mispairing at the cleavage site of Ago2 or some sort of base modification to inhibit Ago2 cleavage). Finally, miRNA activity may also be inhibited using ribozymes instead of antisense RNA. More elaborate details on inhibitors of miRNA can be found in WO2018065390.

miRNA "mimic" is a synthetic nucleic acid molecule designed to interact with endogenous mRNAs. These mimics can comprise nucleic acid analogs, modifications, or comprise artificial double or triple nucleic acid strands. In general, such chemical and/or design modifications are used to enhance the molecule's stability, deliverability, or specificity. Herein nucleotide analog is a nucleotide which contains some type of modification to either the base, sugar, or phosphate moieties. Detailed information is disclosed WO2016106268.

Shadrin et al. (2016) Cell Mol Life Sci 73, 4175-4202 review striated muscle function, regeneration, and repair. Typical examples of muscle defect which can be repaired with cells as obtained in the present are discussed therein and include impaired muscle repair by muscle loss resulting from trauma or large resection and sarcopenia which is characterized by a muscular atrophy, loss of muscle fibers, decreased number and size of motor units, and increased fibrosis and fat accumulation.

Impaired function of cardiac muscle can arise from various congenital or acquired diseases. In ischemic heart disease the buildup of atherosclerotic plaques within coronary arteries increases with age and can cause tissue ischemia or necrosis (myocardial infarction) upon rupture, which can precipitate into congestive heart failure over time. Numerous myopathies, including autoimmune and viral myocarditis, can similarly lead to heart failure if left untreated. Diastolic cardiac dysfunction occurs due to pathological hypertrophy and stiffening of the heart walls that impairs chamber relaxation and filling during the cardiac cycle. The longstanding hypertension, ischemic injuries, aging, and aortic stenosis, as wells as certain metabolic diseases such as glycogen storage disease can all cause diastolic dysfunction. Lastly, congenital heart defects (CHDs) are the most common type of birth defect and represent the leading cause of mortality in infants. Such as ventricular septal defects, tetrology of Fallot, and atrial septal defects .

Since a large number of proteins are expressed in all striated muscles, the same genetic defect can result in both cardiac and skeletal muscle disease. For example, mutations of proteins in the dystrophin-associated glycoprotein complex (DGC) or associated ECM proteins such as laminin and merosin can lead to a wide range of muscular dystrophies. The DGCs link the sarcomeres to the ECM and are involved in the transmission of force and protection of the membrane from shear stress. Thus, dystrophic muscles are damaged more easily, resulting in a repetitive degeneration/regeneration cycles, eventual exhaustion of the SC pool, muscle loss, and fibrosis. Still, not all skeletal muscles are affected equally by muscular dystrophy, with the ocular muscles being typically spared but the diaphragm which is in constant use being the most severely affected muscle and load-bearing muscles such as the gluteus maximus and the posterior muscles of the lower legs also being severely affected. Furthermore, different diseases can affect cardiac and skeletal muscles differently. Duchenne Muscular dystrophy (DMD), the most severe form of muscular dystrophy characterized by the complete loss of functional dystrophin, typically has a more severe skeletal muscle phenotype and a milder cardiac phenotype [89]. In contrast, Becker's Muscular Dystrophy (BMD), characterized by a partial loss of functional dystrophin, has a milder skeletal phenotype and a more pronounced cardiac phenotype.
"treatment" refers to any permanent or temporary amelioration of a symptom of the disorder.

The present invention investigates the regenerative potential of human MiPs *in vivo.* Importantly, it was investigated whether the myogenic propensity of human MAB-MiPs, previously shown *in vitro* [Quattrocelli *et al.* (2015) cited above], was durable *in vivo.* In addition, it was determined whether the human MiPs are necessary for the putative regenerative effect. To address these questions, previously characterized human, isogenic fibroblast-derived MiPs and MAB-MiPs with GFP were equipped, a sodium-iodide symporter (NIS) tracer for non-invasive PET imaging [Holvoet et al. (2015) Stem Cell Reports 5, 1183-1195], as well as an inducible suicidal gene, iCasp9. This iCasp9 gene triggers apoptosis of cells when exposed to the synthetic inducerAP20187 [Xie et al. (2001) Cancer Res 61, 6795-6804]. First, the engineered GFP⁺/iCasp9⁺/NIS⁺ (GIN⁺) MiPs were validated *in vitro.* Suitability for PET imaging was determined by ^{99m}TCO₄ uptake assay. Also, cell death was specifically activated within 48 hours in GIN⁺ cells only after exposure to the inducer, whereas the inducer alone had negligible effect on control cells lacking iCasp9. GIN⁺ MiPs were theni injected in *Rag2*^{*-*/}*⁻;gammac*^{*-*/}*⁻;Sgcb*^{*-*/*-*} mice, which bear skeletal and cardiac muscle degeneration on a xenograft-permissive genetic background [Quattrocelli et al (2015) cited above]. The *Sgcb* model of limb girdle muscular dystrophy was used because it displays more severe phenotype than the *mdx* mouse. Each animal received 5×10⁵ cells as an intramyocardial injection in the left ventricle and during the same procedure 5×10⁵ cells in each femoral artery (bilateral). One week post-injection, GIN⁺ cells were traceable in the heart and in the hindlimb muscles of cell-treated animals, but not of sham-treated, by PET imaging, although some variability in the detection method was observed. Semi-quantitative analysis of the standardized uptake value (SUV) showed no engraftment difference between fibroblast-derived MiPs and MAB-MiPs in the heart, whereas hindlimb muscle engraftment was higher (+38.32%) in MAB-MiP-treated animals (Fig. 1). Four weeks post-injection, half of each cohort received intraperitoneal injection of AP20187, while the other half received a vehicle control. The effect of AP20187 was then investigated 8 weeks after the cell injections. Stereo- and immunofluorescence analyses showed that MAB-MiPs engrafted the heart similarly to fibroblast-derived MiPs. Conversely, MAB-MiPs engrafted the hindlimb muscles more efficiently than fibroblast-derived MiPs. Moreover, detection of engrafted fibers was dramatically reduced afterAP20187 administration. MiP-specific contribution to hindlimb stem cells was quantified by cytometry-based sorting of the GFP⁺ subfraction of resident CD56⁺ satellite cells and AP⁺ MABs. In both pools, MAB-MiP-treated animals displayed larger GFP⁺ subfractions than fibroblast-MiP-treated ones and GFP⁺ cells were undetectable after AP20187 administration.

Differentiation of engrafted MiPs was evaluated using antibodies that detect human but not mouse dystrophin protein (hDYS). hDYS was evident at the sarcolemma following engraftment, and this same pattern was ablated after AP20187 administration. Four weeks post-injection, GFP⁺/hDYS⁺ areas accounted for 34.23±5.96% and 32.68±6.83% of the left ventricular wall in fibroblast-derived-MiP and MAB-MiP-injected mice, respectively (P=0.62, n=5, Mann-Whitney U-test). In the *gastrocnemius* muscles of the same mice, GFP⁺/hDYS⁺ myofibers accounted for 7.25±0.95% and 22.201±5.99% respectively (P<0.05, Mann-Whitney U-test). Myofiber measurement at 8 weeks showed that GFP⁺/hDYS⁺ myofibers accounted for 4,23±1.95% and 14,89±3.73% in fibroblast-MiP- and MAB-MiP-injected mice respectively (P<0.05 Mann-Whitney U-test).

Regeneration of engrafted striated muscles was quantitated by means of tetanic force measurement, treadmill assay, echocardiography and serum creatine kinase (CK) level monitoring. Four and eight weeks post-injection, *extensor digitorum longus* (EDL) tetanic force and run time values on the treadmill showed that MAB-MiP-treated animals performed better than fibroblast-derived-MiP-treated animals. Left ventricle fractional shortening was similarly improved by fibroblast-derived-MiPs as well as MAB-derived-MiPs. Serum CK levels were decreased in MiP-treated animals, with lower levels in MAB-MiPs animals (9.7 ± 0.32 U/I; mean ± s.e.m.-) than in fibroblast-derived-MiP-treated (10, 8 ± 0.21 U/I). Notably, all functional parameters were reverted to baseline-like levels after AP20187 administration. In addition, the morphology of neuromuscular junctions (NMJs) in the skeletal muscles of injected animals was evaluated. When stained with fluorescent bungarotoxin, NMJs in sham animals appeared fragmented, unlike the normal, contiguous structures found in WT muscles. Fibroblast-MiP- or MAB-MiP-engrafted fibers (discriminated from the non-engrafted according to GFP expression) displayed NMJs with similar morphology to WT and strongly reduced fragmentation. Finally, the extent of fibrotic scarring in the skeletal muscle of injected animals was evaluated. Masson's trichromic staining revealed that fibrosis was reduced to a higher extent in MAB-MiP- than in fibroblast-MiP-treated animals, while it appeared partially reconstituted after AP20187 administration. Together, these data indicate that human MiPs have regenerative potential for dystrophic skeletal muscles *in vivo.* Importantly, the reversal of the beneficial effects after AP20187-induced cell death indicates that MiPs are necessary for exerting the observed regenerative effects. Furthermore, the intrinsic *in vivo* propensity towards the skeletal muscle lineage was more evident and durable from MAB-MiPs than those derived from fibroblasts.

To gain insight in the myogenic difference between MAB- and fibroblast-MiPs, it was investigated which genes were differentially expressed between fibroblast- and MAB-MiPs, and whether differentially expressed genes were conserved from the parental cells. To this end, RNA-seq was used to analyze the transcriptional profiles of fibroblast- and MAB-MiPs and the iPSC lines from which the MiPs were derived. For this analysis iPSCs were used at the first stage of differentiation, namely already primed to mesodermal lineages in order to increase the chance of identifying changes in lineage determination genes. The transcriptional profile of both fibroblast-derived- and MAB-derived MiPs was enriched (count >100 FPKM) in genes associated with myogenic mesoderm formation, including the epigenetic regulators *TET1*/*2*/*3, DNMT1*/*3a, HDAC4, SMARCE1*/*2*/*3,* the mesodermal markers *MEF2C, GATA4, TBX3, PAX3, SIX1, ISL1* and the markers of striated muscle *MYOM2*/*3, DMD, SGCB.* Conversely, pluripotency-related genes such as *POU5F1, NANOG, ZFP42, DPPA4, LIN28a, GDF3* were poorly detectable or absent (count <10 CPM; P<0.05 vs all three categories of enriched genes, Wald-log test). Moreover, mRNAs of *AGRIN* and *UTROPHIN* were highly enriched in MiPs (FPKM (avg±s.d.), 1257.15±191.58 and 1560.39±329.15 in fibroblast- and MAB-MiPs respectively). Unbiased sample clustering and principal component analysis showed that samples clustered primarily according to stage (iPSCs vs MiPs), and secondarily according to progeny. Intriguingly, gene ontology (GO) comparison of differentially expressed genes at iPSC stage (fibroblast- vs MAB-iPSCs) and MiP stage (fibroblast- vs MAB-MiPs) showed high overlap (79.53%) of Biological Process terms. Overlapping GO terms mainly pertained to developmental program, signaling and cellular metabolism. When clustered on a heat map, patterns of gene expression emerged reflecting stage-specific (differing iPSCs from MiPs, regardless of progeny) or progeny-specific (differing fibroblast- vs MAB-derived cells, regardless of stage).

905 genes were found to be differentially expressed between fibroblast-derived-MiPs and MAB-MiPs. Among the significantly differentially expressed genes (P_{adj} <0.05), several myogenic inhibitors were upregulated in fibroblast-derived-MiPs, whereas several muscle proteins and myogenesis-associated genes were upregulated in MAB-MiPs. Agonists of BMP signaling such as *BMP6, SMAD5* and *LTBP4* were upregulated in fibroblast-MiPs, whereas the BMP signaling inhibitor *SMAD7* was upregulated in MAB-MiPs (Fig. 2). Plotting the fold change of significantly differentially expressed genes in MiPs compared iPSCs, 56.17% of differentially expressed genes conserved the progeny-specific trend across stages, including many identified with the previous analysis. A subset of genes important for myogenesis was validated using qPCR. The expression levels by qPCR between fibroblast- and MAB-progenies were compared at somatic, iPSC and MiP stages. *OSTN, MYB, LHX2, BMP6* and *SMAD5* were consistently upregulated in fibroblasts, fibroblast-iPSCs and fibroblast-MiPs, while *ANXA3, SMAD7* and *PAX7* were upregulated in MABs, MAB-iPSCs and MAB-MiPs. *LTBP4* was upregulated in fibroblasts and fibroblast-MiPs, but not in fibroblast-iPSCs, and similarly *ANXA7* was upregulated in MABs and MAB-MiPs, but not in MAB-iPSCs.

Then CpG methylation and histone mark enrichment were examined in the promoters of these same genes. Bisulfite sequencing analyses revealed that CpG methylation for *OSTN, MYB, LHX2, BMP6* and *SMAD5* was increased in the MAB progeny, while CpG methylation for *ANXA3, SMAD7* and *PAX7* was increased in the fibroblast progeny. The CpG methylation patterns appeared less discriminative for *LTBP4* and *ANXA7.* Chromatin immunoprecipitation analyses revealed that the non-permissive marker H3K9me3 was correlated with the methylation patterns and, conversely, the permissive markers K4me2 and K27ac correlated with the transcriptional upregulation trends. Notably, *LTBP4* showed enrichment of K9me3 in the MAB progeny, of K4me2 in fibroblasts and fibroblast-MiPs, and of K27ac in fibroblast-iPSCs, MAB-iPSCs and MAB-MiPs. Conversely, ANXA7 showed enrichment in K9me3 in the fibroblast progeny and in permissive marks in the MAB progeny, with a spike of K27ac in fibroblast-iPSCs. In association with the myogenic propensity shown by MiPs *in vivo, MYB, LHX2, LTBP4, BMP6* and *SMAD5* were defined as an anti-myogenic gene pool, and *ANXA3*/*7, SMAD7, PAX7, CAV1* and *SIRT1* as a pro-myogenic gene pool.

It was investigated whether perturbation of these pools could shift the myogenic propensity of fibroblast- and MAB-MiPs. To this end, the endoribonuclease-prepared small interfering RNAs (esiRNAs) targeting the anti-myogenic pool were combined in a pro-myogenic cocktail. Conversely, the esiRNAs targeting the pro-myogenic pool were combined in the anti-myogenic cocktail. Scramble esiRNAs were used as control conditions. Since several key components of the BMP cascade were involved in both gene pools, the anti-myogenic cocktail was enhanced with soluble BMP6 and the pro-myogenic cocktail with soluble Noggin. First, the effects of anti-myogenic and pro-myogenic cocktails were validated on target gene expression. Anti-myogenic pool genes, *LHX2, LTBP4, MYB, OSTN,* and SMAD5, were downregulated in the presence of pro-myogenic cocktail. Similarly, the pro-myogenic pool genes, including *ANXA3, ANXA7, PAX7,* and *SMAD7,* were downregulated in the presence of anti-myogenic cocktail. Then the effect of these cocktails was tested on *in vitro* myogenic propensity of MiPs in co-culture with C2C12 myoblasts and stained with lamin A/C human nuclei in chimeric myotubes. A significant increase (P<0.05 vs own ctrl (scramble), 2-way ANOVA, Kruskal-Wallis test) saw increased in the number of myofibers with 3 human nuclei after PMC treatment compared to cells that received the AMC treatment or to controls. Additionally, the co-cultures were also performed in the presence of the suicidal gene in the MiPs, GIN⁺ MiPs. A higher myogenic propensity would result in higher contribution to chimeric myotubes and hence a higher loss of myotubes after exposure to AP20187. Myogenic propensity was significantly reduced (P<0.05 vs own ctrl (scramble), 2-way ANOVA, Kruskal-Wallis test) in fibroblast-derived MiPs and MAB-MiPs after treatment with anti-myogenic cocktail, whereas it was increased in pro-myogenic cocktail treated cells. Importantly, pro-myogenic cocktail-treated fibroblast derived-MiPs showed comparable myogenic differentiation to control MAB-MiPs. Thus, perturbation of gene subsets by means of defined factor cocktails enabled perturbation of MiP myogenic propensity. Particularly, reduction of anti-myogenic genes in fibroblast-derived- MiPs resulted in a myogenic potential that is comparable to MAB-MiPs.

In light of retained miRNA signatures after reprogramming [Vitaloni et al (2014) cited above], it was investigated whether miRNAs also influenced the myogenic potential of MiPs. The miRNA component of the same samples was analysed that were previously analyzed by RNA-seq. The miRNA profile of both fibroblast derived and MAB MiPs was enriched (count >30 FPKM) in miRNAs associated with mesodermal progression and myogenesis including *let-7a, miR-1*/*-590*/*-497*/*-34a*/*-27b*/*-101*/*- 133a*/*-138*/*-15a*/*-15b*/*-16*/*-199a*/*-21*/*-22*/*-221*/*-23a*/*-24.* In contrast, miRNAs associated with pluripotency including *miR-372*/*-302a*/*-302b*/*-302c*/*-302d*/*- 367*/*106a*/*-363* were barely detectable or absent in these cells (count <2 FPKM). Similar to the observations in the RNA-seq dataset, unbiased sample clustering and principal component analysis showed stage-specific clustering (iPSCs vs MiPs), with cell type-specific segregation (fibroblast-derived vs- MAB-derived) among each stage. At the MiP stage, 611 differentially expressed miRNAs were found discriminating fibroblast derived-MiPs versus MAB-MiPs. Among the significantly upregulated miRNAs in fibroblast-MiPs, those with predicted targets among the upregulated genes previously identified by RNA-seq were selected. However, it was not experimentally confirmed that selected miRNAs are directly targeting the selected differentially expressed genes. Data on the 3'UTR binding, prediction and related mirsvr scores were obtained from microRNA.org (Fig. 5). Following this RNA-seq-based filter, *miR-34c-5p*/*34c-3p*/*-362*/*-210*/*-590* were identified for fibroblast derived-MiPs, and *miR-212*/*-132*/*-424*/*-146b*/*-181a* for MAB-MiPs (Fig. 3 and Fig. 5). Among those, *miR-34c-5p*/*-34c-3p*/*-362* and *miR-132*/*-424*/*-146b* followed the same differential expression trend seen at the iPSC stage, together with 44.84% of total differentially expressed miRNAs (Fig. 4). The expression profile of all these miRNAs were validated across somatic, iPSC and MiP stages using qPCR. As in the previous experiments, it was the aim to define oligonucleotide cocktails to simultaneously perturb these miRNAs. Synthetic inhibitors of fibroblast-MiP-associated miRNAs were combined with synthetic mimics of MAB-MiP-associated miRNAs and tested whether this had pro-myogenic potential. Conversely, inhibitors of MAB-MiP-associated miRNAs and mimics of fibroblast-MiP-associated miRNAs were tested for anti-myogenic potential. These inhibitor and mimics were tested for their effect on target miRNA level expression. Notably, MiPs treated with this miRNA-based anti-myogenic cocktail showed downregulation of pro-myogenic genes. Those MiPs treated with a miRNA-based pro-myogenic cocktail had decreased levels of anti-myogenic genes (Fig. 8). Consequently, it was investigated whether the miRNA-targeting cocktails could shift the myogenic propensity of MiPs when co-cultured with C2C12. It was found that miRNA based anti-myogenic cocktail decreased myotube formation while the miRNA-based pro-myogenic cocktail increased the myogenic differentiation. The cocktails were tested in presence and in absence of the apoptotic drug (Fig. 6). A significant (P<0.05 vs own ctrl (scramble), Kruskal Wallis) upregulation was observed of chimeric fibers containing 3 or more human nuclei in co-cultures that received pro-myogenic treatment compared to cells that received the anti-myogenic treatment or to controls. Finally, the translational relevance of such approach was tested by injecting cocktail-pretreated cells in the femoral artery of *Rag2*^{*-*/}*⁻;gammac*^{*-*/}*⁻;Sgcb*^{*-*/*-*}mice. Four weeks post-injection, MiP-specific engraftment and hDYS expression appeared significantly decreased (P<0.05 vs own ctrl (scramble), Kruskal Wallis) for anti-myogenic-treated cells and increased for pro-myogenic-treated cells, when compared to relative controls. Quantitating the regeneration levels by means of hDYS protein levels, it was found that pro-myogenic-treated fibroblast-MiPs performed as control MAB-MiPs (Fig. 7). Thus, it has been shown that a defined combination of microRNAs and soluble ligands contributes to somatic lineage determination in MiPs. Furthermore, perturbation of defined subsets of miRNAs by means of oligonucleotide cocktails was able to rescue the myogenic potential gap observed in fibroblast-MiPs compared to MAB-MiPs.

Next, in order to unravel additional potential targets of the microRNAs, RNA-seq analysis was performed after the treatment with the selective miRNA cocktails Cells clustered according to the treatment that was conducted. 6000 genes were differentially regulated between MAB-MiPs treated with pro-myogenic cocktails and untreated, and 3736 genes differentially expressed between MAB-MiPs treated with anti-myogenic vs untreated. For the fibroblast-MiPs 1194 differentially expressed genes were detected between pro-myogenic treated cells and untreated, and 829 between anti-myogenic treated cells and untreated (P_{adj} <0.05, Wald-log test). Consistent with previous analysis *ANXA3* was upregulated in MAB-MiPs upon pro-myogenic treatment, while component of the BMP cascade, such as *SMAD9* and *BMP6* were downregulated in MAB-MiPs treated with pro-myogenic and upregulated in MiPs treated with anti-myogenic cocktails. *LTBP4* was also upregulated in cells treated with anti-myogenic cocktail, whereas MYB was downregulated upon pro-myogenic treatment. In addition, genes encoding for muscle proteins, including *MYL6, ACTA2, TNNT1,* were upregulated in pro-myogenic-cocktail treated MiPs, and conversely downregulated in anti-myogenic treated. In these cells myogenic promoters and gene critical for muscle regeneration such as *HAND1, CXCL16, MET* and *DLL* were found downregulated. Interestingly, genes involved in smooth muscle differentiation were observed, including *CNN2* and *CNN3* and other genes relevant for skeletal muscle functionality like *MAST1* significantly downregulated in cells that received an anti-myogenic treatment. Epigenetic regulators, including *TET1, CBX6* and *HDAC6* were found differentially regulated following the miRNA treatment. Furthermore, in cells treated with anti-myogenic cocktails upregulation of several genes were detected that could be involved in the muscle homeostasis, TGFB ligand *GDF15* and autophagy related genes *ATG10* and *ATG14*

Finally, the CpG methylation and the histone markers enrichment were investigated in the promoters of the putative pro-myogenic gene polls and anti-myogenic gene pools. In accordance to the RNA-seq data anti-myogenic genes *BMP6, SMAD5* and *MYB* showed a decrease in methylation in MAB-MiPs treated with anti-myogenic cocktail. Furthermore, in these cells *MYB* displayed an increase in permissive histone markers H3k4me2, H3k27ac. When treated with pro-myogenic cocktails, MAB-MiPs showed a decrease of the non permissive marker H3k9me3 in *ANXA3* as well as an enrichment of permissive histone marker H3k4me2 in the promoters of pro-myogenic genes *PAX7, ANXA3* and *SMAD7.*

In fibroblast-MiPs methylation was increased in the promoters of *SMAD7* and *ANXA7* upon anti-myogenic treatment and this correlates with an increase in H3k9me3 in the promoters of these genes. Conversely anti-myogenic genes such as *MYB* and *BMP6* were found partially methylated and decreased in permissive markers H3k4me2 and H3k27ac after treatment with pro-myogenic cocktail in accordance to what was observed in the RNA-seq data. Taken together the present results provide more insight on the anti-myogenic and pro-myogenic miRNA cocktails that were previously defined, adding additional interesting targets that could be responsible for the differential *in vivo* performance of MiPs.

Simultaneous regeneration of skeletal and cardiac muscle in dystrophic subjects is compelling, considering that effective repair of skeletal muscle would likely worsen heart conditions [Townsend et al (2008) Mol Ther 16, 832-835]**.** In this regard, MiPs may represent a valid cell tool, as they can be injected in the circulation and efficiently regenerate both striated muscle types [Quattrocelli et al (2015) cited above]. However, two main questions remained unaddressed with respect to the actual translational potential of this iPSC-based strategy: *in vivo* behavior of human MiPs and whether the myogenic differentiation potential of human MiPs is influenced by reprogrammed cell types or origins, and if external modulators could improve the performance.

To this end the *in vivo* potential of human MiPs in immunodeficient dystrophic mice was explored in order to determine whether the source of MiPs alters the outcome after engraftment. Taking histological, molecular and functional data together, the capacity to regenerate skeletal muscle of MAB-MiPs appears greater than fibroblast-derived-MiPs. Interestingly, the cardiomyogenic potential seems comparable between the two MiP types. These features recapitulate the *in vitro* behavior previously shown for those cells [Quattrocelli *et al.* (2015) cited above]. In order to document that improvement after engraftment was due to cell intrinsic effects, induced apoptosis in engrafted MiPs was used and it was found that this associated with reversal of the beneficial effects at both molecular and functional levels. It cannot be excluded that the detrimental effects were partially linked to the drug that induced apoptosis, AP20187. However, this seems unlikely considering that the compound has negligible toxic effects *in vivo* below 10mg/kg [Jin et al. (2000) Nat Genet 26, 64-66]. Intriguingly, MiP engrafted fibers showed improved NMJ morphology to levels comparable to WT fibers. The mRNAs for AGRIN and UTROPHIN, reported agonists of NMJ formation [Zhang et al. (2015) Dev Neurobiol 76, 551-565; Adams et al. (2010) J Neurosci 30, 11004-11010], were highly enriched in MiPs. More refined studies are still needed to address the mechanism by which MiPs regenerate the fragmented NMJs in engrafted skeletal muscle fibers.

In this study, dual delivery was used by injecting into both the heart and femoral arteries to remain consistent with previously reported conditions in mice [Quattrocelli *et al.* cited above]. However, only the mechanisms to enhance the myogenic potential of MiPs were further explored, given the different performance *in vitro* and *in vivo* of MiPs towards skeletal muscle, dependent on their cell type or origin, while the cardiac commitment *in vivo* did not show differences between human f- and MAB-MiPs, consistently with murine and canine MiPs [Quattrocelli *et al.* (2015) cited above]. Analysis of transcriptional profiles in MiPs confirms that MiPs retain much of the identity of their original cell source. Genes to be manipulated to enhance myogenic potential were selected based on their known involvement in MD or in muscle biology. Both *LTBP4,* which was upregulated in fibroblast-MiPs, and *ANXA7* which was upregulated in MAB-MiPs, showed progeny-specific differential expression in somatic cells, but not in iPSCs. Considering the epigenetic data, it appears that both genes presented a rather permissive histone signature in these iPSCs. This probably contributed to restoring similar expression levels of *LTBP4* and *ANXA7* in fibroblast- and MAB-iPSCs. Therefore, it may be possible that the progeny-related trends of differential expression for these genes are linked to a non-pluripotent state. *LTBP4* is a TGFbeta regulator in the skeletal muscle, and can modify muscular dystrophy [Heydemann et al. (2009) J Clin Invest 119, 3703-3712]**.** Intriguingly, annexins are also being investigated as genetic modifiers of dystrophic progression [Swaggart et al. (2014) Proc Natl Acad Sci USA 111, 6004-6009]. In the present experiments, *LTBP4* appeared associated with decreased myogenic potential, while *ANXA7,* together with *ANXA3,* associated with increased propensity. Thus, more focused efforts should be directed on how these factors possibly modify the lineage potential of human MiPs.

Use of miRNAs for enhancing myogenic fate *in vivo* is particularly compelling and may have high translational potential [Li & Rana (2014) Nat Rev Drug Discov 13, 622-638]. To address whether miRNAs could be identified that could be used to promote myogenesis, RNA-seq and miRNA-seq data were overlaid with the aim of defining key components that could be used to enhance myogenic potential. In both RNA- and miRNA-seq, the samples cluster primarily according to cell type and this is reflected in the iPSCs and their resulting MiPs. However, MiPs, whether derived from fibroblast or MABs, share many differentially expressed genes but fewer miRNAs. This observation indicate that MiPs may require a more complex body of miRNAs to tightly control a smaller transcriptional divergence. Based on parental cell progeny, combinations of miRNAs were defined to modulate MiP myogenic propensity. Myogenic differentiation *in vitro* and *in vivo* was respectively decreased or increased using cocktails of miRs. Notably, a cocktail of pro-myogenic miRs was able to rescue fibroblast-derived MiPs to the degree that they performed similarly to MAB-MiPs. Intriguingly, among the miRNAs that were filtered into the anti-myogenic pools, *miR-34c*/*-362*/*-210* have been previously associated with pathological state of muscles [Roberts et al. (2012) Mol Ther Nucleic Acids 1, e39; Dmitriev et al. (2013) J Biol Chem 288, 34989-35002; Endo et al. (2013) Biol Pharm Bull 36, 48-54] and *miR-590* has been recently associated with differentiation inhibition and the TGFbeta pathway [Liu et al. (2015) Mol Med Rep 12, 7403-7411]**.** Conversely, within the pro-myogenic pool, *miR-424*/*-146b*/*-181a* are associated with myogenesis and muscle development [Wang et al (2014) Muscle. PLoS One 9, e96857; Fiorillo et al. (2015) Cell Rep 12, 1678-1690; Naguibneva et al. (2006) Nat Cell Biol 8, 278-284]. *miR-212*/*-132* have been shown to inhibit *MECP2* [Mayer et al. (2015) Circ Res 117, 622-633], which in turn regulates muscle maturation [Conti et al. (2015) PLoS One 10, e0130183]. The miRNAs tested in the cocktails were selected based on their known functions as well as their differential expression.

RNA-sequencing analysis after anti-myogenic and pro-myogenic miRNA treatment allowed identifying target genes involved in miRNA regulation of MiPs. A high number of genes were found to be differentially expressed upon miRNA cocktail exposures. Among those, *ANXA3, SMAD5, BMP6, MYB, LTBP4* were predicted targets of miRNAs included in the cocktails. In particular exposure to pro-myogenic cocktail determined a consistent downregulation of elements of the BMP/TGFβ signaling pathway, and conversely anti-myogenic exposure resulted in an increased of the TGFβ agonist *GDF15,* that has been interestingly associated with muscle wasting *in vivo* [Patel et al. (2016) J Cachexia Sarcopenia Muscle 7, 436-448]. Upon pro-myogenic treatment upregulation was detected of genes coding for proteins important for skeletal *(ACTA2, ABLIM3)* and smooth (*CNN2, CNN3*) muscle homeostasis. These findings indicate potential contributions of the pro-myogenic miRNAs that were selected in enhancing smooth muscle differentiation as well thus opening the research of MiPs for appealing new strategies for muscle degenerating disease like MDs, where smooth muscles are highly affected. Consistent with the *modus operandi* of miRNAs many epigenetic modulators were found differentially regulated in treated cells. Interestingly An upregulation of *SAFB2* was detected in pro-myogenic treated cells. It has been shown that its paralog *SAFB1* facilitates the transition of myogenic gene chromatin from a repressed to an activated state [Hernández-Hernández et al. (2013) Nucleic acids research 41, 5704-5716]; The present results indicate that *SAFB2* could be involved in similar processes. Finally, upregulation of autophagic markers was detected such as *ATG10* following anti-myogenic treatment, whose aberrant expression has previously been implicated in disease associated with atrophy of the skeletal muscle [Rusmini et al. (2015) Sci. reports 5, 15174].

Moreover, CpG island methylation and histone markers enrichment analysis after miRNA treatment shed more light on the set of anti-myogenic and pro-myogenic genes that were put forward. Although CpG island methylation analysis was conducted on the most proximal CpG islands to the transcription start site, this does not indicate that they are directly implicated in gene regulation, nor that they are the only CpG islands responsible for it. Nevertheless, the present data indicate that these genes might be epigenetically regulated at the same time by miRNAs, DNA methylation and histone modifications.

In particular anti-myogenic BMP6, *SMAD5* and *MYB* displayed a consistent pattern of DNA methylation and histone markers enrichment, indicating a simultaneous multifactorial regulation. Other genes, such as *ANXA3* and *LTBP4* did not show univocal signature at epigenetic level upon treatment. Finally, *ANXA7, PAX7* and SMAD7 promoters' methylation and histone markers enrichment was modulated upon treatment, although they were not detected among the differentially expressed genes, indicating once again the multifactorial mechanisms of actions of epigenetic regulators.

miRNA-based strategies as disclosed in the present invention can benefit muscle regeneration not only based on cell delivery, but also mobilizing and modulating resident stem cells.

Large animal models, are available for scaling up. To this end the Golden Retriver Muscular Dystrophy model (GRMD) is used to investigate cell engraftment, dystrophin restoration and functional rescue of both striated muscle types.

### Examples

### Example 1. Injection of MiPs and animal models

Human fibroblast- and MAB-MiPs derived from fetal fibroblasts and MABs were transduced with GFP-iCasp9- and NIS-Puro^{R}-bearing viral vectors [Quattrocelli et al (2015) cited above; Straathof et al. (2005) Blood 105, 4247-4254; Holvoet et al. (2015) Stem Cell Reports 5, 1183-1195.]. GIN+ cells were then sorted for GFP and cultured in the presence of 1µg/ml puromycin (Sigma-Aldrich), following reported conditions as disclosed in [Quattrocelli et al (2015) cited above]. All protocols on live mice were performed in compliance with the Belgian law and the Ethical Approval of KU Leuven (P095/2012).

*Rag2-null*/*gammac-null*/*Sgcb-null* male mice were divided in randomized groups at 3 months of age (n=6, sham; n=6, fibroblast-MiPs; n=6, MAB-MiPs) and injected with GIN⁺ MiPs (passage 7-10). MiPs were exposed to RPMI20%10% medium for48hours, then injected in parallel in the left ventricle myocardium and in both femoral arteries under isofluorane anesthesia into each animal (5×10⁵ cells/5×2.5µl in the myocardium; 5×10⁵ cells/100µl per femoral artery). Sham-treated controls received equal treatment and amounts of cell-free saline solution. Engraftment, regeneration and functional outcome were investigated at 4 and 8 weeks post-injection. One day after the mid-term analyses, 3 mice from each cohort were injected with AP20187 (5x2mg/body-kg every other day, i.p.), whereas the other mice of each cohort received vehicle injections.

High-resolution digital ultrasound images were obtained by an experienced echocardiographer using Vevo 2100 Imaging System (Visualsonics) with a 30 MHz probe. Mice were anaesthetized using 1% isofluorane in oxygen, and positioned on the heating pad of the system, in order to maintain normothermia under continuous monitoring. Pre-warmed ultrasound gel was applied on the shaved thorax. B-mode-based 3D reconstruction was carried using the VisualSonics rail system with fixed probe, with ECG- and respiratory gating. FS was calculated based on LVIDd/s values, whereas EDV and CO were calculated based on 3D analysis. Raw data were collected in blind.

Treadmill analysis was conducted on a 10°-uphill oriented treadmill belt with 1m/min² acceleration on a starting speed of 10m/min. Mice run was stopped after ≥5 consecutive seconds on the pulsed grill.

Muscle force assessment was performed on freshly isolated EDL muscles upon sacrifice, using a 1200A *in vitro* muscle test system (Aurora Scientific). Muscle force was probed upon 20 iterated bouts of isometric contractions (200hz, 80V, 0.5msec stimulation, 0.5sec tetanus, 10sec interval; 30°C) in dedicated buffer (1.2mM KH₂PO₄, 0.57mM MgSO₄^{∗}7H2O, 2mM CaCl₂^{∗}2H₂O, 10mM HEPES, 0.5mM MgCl₂^{∗}6H₂O, 0.5mM MgCl₂^{∗}6H₂O, 4.5mM KCl, 120mM NaCl, 0.7mM Na₂HPO₄, 1.5mM NaH₂PO₄, 10mM D-Glucose, 15mM NaHCOs; pH 7.3; Sigma-Aldrich). Data were analyzed as % of max absolute force of input sham muscles.

CK levels were measured in resting conditions (>24hours after last treadmill exercise) from serum obtained from >50ul blood (withdrawn from the tail vein). CK level quantitation was performed using the Creatine Kinase Activity Colorimetric Assay kit (BioVision), following manufacturer's instructions for both sample preparation and standard curve assessment.

### Example 2. Cell differentiation

MiP differentiation with C2C12 myoblasts was conducted as disclosed in Quattrocelli et al (2015) cited above. Briefly cells were seeded 1:10 MiP/myoblast ratio in RPMI 20%/10% medium on collagen-coated vessels for 24 hours, then differentiated in DMEM 2% Horse serum medium for 96 to 120 hours in 5% O₂/5% CO₂ at 37°C. MiPs and C2C12 were seeded and AP20187 (10nmol) was added 48 hours prior to immunostaiing analysis. Myogenic differentiation of human MiPs was conducted seeding 5,000 cells/cm² on gelatin(Millipore)-coated plastic (NUNC) in DMEM-F12 supplemented with 20% FBS and 1% ITS (all reagents from Thermo Fisher Scientific). After 24 hours, medium was changed to DMEM-F12 supplemented with 20% FBS, 2nM SB431542 hyclate and 2nM LDN193189 hydrochloride (Sigma-Aldrich). After 48 hours, medium was changed to DMEM-F12 supplemented with 2% horse serum, 2nM SB431542 hyclate and 2nM LDN193189 hydrochloride for additional 48 hours, prior to immunostaining analysis.

Gene-targeting cocktails were composed as follows: AMC, esiRNAs anti-ANXA3/- ANXA7/-PAX7/-SMAD7 (Sigma-Aldrich); PMC, esiRNAs anti-OSTN/-MYB/-LHX2/- BMP6/-SMAD5/-LTBP4 (Sigma-Aldrich). Cells were transfected with a total of 1mg esiRNAs and 1ml lipofectamine 2000 (Thermo Fisher Scientific) per mw24 well. AMC-treated cells were then kept in medium supplemented with 100ng/ml BMP6 (Peprotech), whereas PMC-treated cells in medium supplemented with 100ng/ml Noggin (Thermo Fisher Scientific). Gene expression and differentiation assays were conducted 48 hours after transfection. miRNA-targeting cocktails were composed as follows: AMC, miR-mimics for miR-34c-5p/-34c-3p/-362/-210/-590, anti-miRs anti-miR-132/-146b/-424/-212/-181a; PMC, miR-mimics for miR-132/-146b/-424/-212/- 181a, anti-miRs anti-miR-34c-5p/-34c-3p/-362/-210/-590 (all oligonucleotides from Sigma-Aldrich). Cells were transfected with 100nmol of each miR-mimic and 20nmol of each anti-miR per mw12 well, with 2µl lipofectamine 2000. Gene/miRNA expression and differentiation assays were conducted 48 hours after transfection.

### Example 3. Non-invasive imaging

Wild type and GIN⁺ MiPs were plated in quadruplet and incubated with pertechnetate (^{99m}TcO₄⁻) tracer solution (0.74 MBq/ml in DMEM) for 1 hr. afterwards, cells were rinsed with ice-cold PBS and supernatant was collected. The cells were lysed and collected. The radioactivity of the pellet and supernatant was measured by 2480 Wizard² Automatic Gamma Counter (PerkinElmer, Waltham, MA, USA). The results were adjusted for tracer decay. Uptake values were corrected for cell amounts in the according samples as measured via the NucleoCounter NC-100 system (ChemoMetec, Allerod, Denmark). The mice received an intravenous injection of 3.7-5.55 MBq ¹²⁴I (PerkinElmer) on day 3 and an intramuscular injection of 100 µl LASIX (20 mg/ml, Sanofi, Paris, France) as diuretic. At 3 hrs later, a 20 min static scan was acquired with the Focus 220 small-animal PET system (Siemens Medical Solutions, Malvern, PA, USA). A transmission scan was acquired using a ⁵⁷Co source (185 MBq, Eckert and Ziegler, Berlin, Germany). PET images were reconstructed using a maximum a posteriori (MAP) image reconstruction algorithm and were then analyzed with PMOD 3.0 (PMOD technologies, Zurich, Switzerland) Data were averaged on both legs per animal to keep the intra-injection variability in account. SUV was calculated according to the following formula: SUV=activity concentration in volume of interest/(injected activity/weight of animal). Volumes of interest were manually positioned around the graft regions.

### Example 4. Molecular and immunostaining assays

Validation of RNA-seq and miRNA-seq data was carried out by means of qPCR, using SybrGreen for gene levels and Taqman for miRNA levels. Gene qPCR was performed on 1:5 diluted cDNA obtained from 1µg total RNA (SybrGreen mix, SSIII cDNA production kit and RNA extraction kit from Thermo Fisher Scientific), using Viia7 384-plate reader (Thermo Fisher Scientific; final primer concentration, 100nM; final volume, 10µl; *PGK,* internal reference; thermal profile, 95°C 15sec, 60°C 60sec, 40x). miRNA qPCR was performed on 1:15 diluted cDNA obtained from 20ng total miRNA preparation (miRNA isolation kit, Thermo Fisher Scientific). Reagents and probes for reverse transcription and Taqman-based qPCR are from Thermo Fisher Scientific, and manufacturer's protocols were applied.

Methylation patterns were assayed by means of bisulfate sequencing of CpG islands in the promoter regions of target genes (as reported in UCSC genome browser (hg19); primers by MethPrimer). Genomic DNA was isolated through genomic DNA mini kit (Thermo Fisher Scientific), then 1µg/20µl was bisulfate-converted using EpiTect Bisulfate kit (Qiagen). Single CpG island amplicons were amplified by PCR (final primer concentration, 330nM; final volume, 20µl; thermal profile: 95°C 30sec, 55°C 60sec, 72°C 60sec, 40x) at T3000 thermocycler (Biometra) using Taq polymerase (Thermo Fisher Scientific), then gel-extracted by means of Gel Extraction kit (Thermo Fisher Scientific) and ligated into pGEM plasmids via TA cloning (Promega). Single bacterial clones were bulk-sequenced (GATC Biotech) and analyzed by means of QUMA online software. Statistical analysis was performed on average methylation values of 5 sequences per cell clone (3 clones per cell type, from independent donors).

Histone mark levels were assayed by means of chromatin immunoprecipitation (ChIP) on the same CpG islands assayed by bisulfate sequencing, adapting previously reported conditions to 5×10⁶ cell pellets [Carey et al. (2009) Cold Spring Harb Protoc pdb prot5279]. 1µg/10µgDNA polyclonal antibodies anti-K9me3 (repressive mark), anti-K4me2 (permissive mark) and anti-K27ac (active mark; all antibodies from Active Motif #39142/39133/39765) was used in the ChIP, and protein-A-coated sepharose beads (GE Healthcare) were used for the subsequent pull-down. IgG isotype (eBioscience) was used as negative ChIP control. 5µl out of 100µl initial sonicated genomic DNA fragment suspension was used as reference input. Purification of ChIP and input DNA was performed by means of MinElute kit (Qiagen) and quantification as % of input was performed through SybrGreen qPCR, following conditions reported above. Statistical analysis was performed using 3 qPCR replicates per cell clone (3 clones per cell type, from independent donors).

Western Blot (WB) analyses were performed on 50µg cell/tissue lysate (100µg for DYS analysis) according to commonly used procedures in 10% acrylamide (6% for DYS analysis) hand-cast gels. Here follows the list of antibodies and relative dilutions: mouse anti-sarcomeric α actinin (Abcam #ab72592), 1:500; rabbit anti-GFP (Thermo Fisher Scientific, #A11122), 1:500; mouse anti-DYS3 (interacting with canine and human isoforms, Novocastra #DYS3-CE-S), 1:500. Bands were detected and pictured at Bio-Rad GelDoc by means of Pico substrate (Thermo Fisher Scientific; Dura substrate for DYS analysis). Densitometric analyses were carried on gels loaded, blotted and detected in parallel by means of QuantityOne software (Bio-Rad).

Whole fluorescence imaging of injected tissues was performed at Olympus SZX12 stereomicroscope by means of SISgetIT software (2" exposition for GFP, 0.2" for brightfield, semirefringent bottom) murine MiP *in vivo* experiments, and with Zeiss SteREO Discovery V12 microscope by means of AxioImaging software (2" exposition for GFP, 0.2" for brightfield, semirefringent bottom). For NMJ imaging and quantitation, mouse soleus and extensor digitorum longus muscles were dissected and fixed in 4% PFA. Muscle fibers were prepared and stained with rhodamine-coupled bungarotoxin using 1:2.500 dilution (rhodamine-BTX, Invitrogen) for 1 h at room temperature. Stained bundles were washed three times and embedded in Mowiol. Z-stacks of individual NMJs were taken with 40x oil objective (Zeiss Examiner Z1). Images were deconvoluted and analysed using 3D deconvolution and 3D measurement modules in AxioVision Software. Data are presented as the mean values, and the error bars indicate ±s.e.m. The number of biological replicates per experimental variable (n) is usually n>5 or as indicated in the figure legends. The significance is calculated by unpaired two-tailed t test and provided as real p-values that are believed to be categorized for different significance levels, like,*** p <0.001, ** p <0.01, or * p <0.05. Immunofluorescence staining was performed following the commonly used steps of Triton-based (Sigma-Aldrich) permeabilization, donkey serum-(Sigma-Aldrich) background blocking, overnight incubation with primary antibody at 4°C, 1hour incubation with 1:500 AlexaFluor-conjugated donkey secondary antibodies (Thermo Fisher Scientific), and final counterstain with Hoechst. Here follows the list of primary antibodies and relative dilutions: rabbit anti-laminin (Sigma #L9393), 1:300; goat anti-GFP (Abcam #ab5450), 1:500; mouse anti-MyHC (DSHB #MF20), 1:3; mouse anti-sarcomeric α actinin (Abcam #ab72592), 1:300; rabbit anti-Myocd (SantaCruz #sc-33766), 1:100; mouse anti-DYS3 (canine- and human-specific, Novocastra #DYS3-CE-S), 1:100; mouse anti-Pax3 (R&D #MAB2457), 1:300; rabbit anti-lamin A/C (#Epitomics 2966-1), 1:600. Imaging was performed at Eclipse Ti microscope (Nikon) by means of Image-Pro Plus 6.0 software (Nikon). Quantitation of engraftment, satellite cell and fiber counts was performed by means of ImageJ software (NIH, USA) on at least 10 fields across heart and muscle samples. SCs and MABs were sorted as CD56⁺ and AP⁺ populations at passage 0. Antibodies: mouse anti-CD56 (R&D, FAB7820A), 2µl/10⁵cells; mouse anti-AP (R&D, #FAB1448P), 2µl/10⁵cells.

### Example 5. RNA-seq and miRNA-seq

A 10×10⁶ cell pool per clone was divided in two 5×10⁶ pools for RNA (Total RNA isolation kit and post-isolation DNase, Thermo Fisher Scientific) and miRNA (miRNA isolation kit, Thermo Fisher Scientific) extraction, respectively. RNA (>10µg) and miRNA (approximately 1µg) samples were then verified and processed by the Genomics Core (KU Leuven - UZ Leuven, Belgium). RNA sequencing libraries were constructed with the TruSeq RNA Sample Prep Kits v2 (Illumina). RNA-sequencing after miRNA treatment was performed 48 hours after treatment (n=3 per conditions). Three RNA samples per group (one per clone) were indexed with unique adapters and pooled for single read (50bp) sequencing in Illumina HiSeq2000. RNA-seq reads were aligned with TopHat v2.0.2 to the human genome version hg9 [Trapnell et al. (2012) Nat Protoc 7, 562-578]. Transcripts were assessed and quantities were determined by Cufflinks. Differential expression levels were assessed using DESeq [Anders et al. (2013) Nat Protoc 8, 1765-1786]. GO analysis was performed by means of BinGO (biological process; within Cytoscape 3.2.1) on DE genes in fibroblast- vs MAB-iPSCs, and in fibroblast- vs MAB-MiPs comparisons. GO terms with P<0.05 were subsequently compared between iPSC and MiP stages. Variance, count and fold change analyses, as well as charts and z.test matrices were conducted by means of Excel software (Microsoft). Heat maps were obtained analyzing the z.test value matrices with GITools 2.2.2 (hierarchical clustering, Manhattan distance, average linkage) [Perez-Llamas (2011) PLoS One 6, e19541]. Data about 3'UTR binding prediction and related mirsvr scores were obtained from microRNA.org [Betel et al. (2008) Nucleic Acids Res 36, D149-153; Betel et al. (2010) Genome Biol 11, R90](Aug 2010 release; conserved, good-score matrix).

### Example 6. Statistical analyses

Sample size for in *vitro*/*in vivo* experiments was calculated by means of Sample Size Calculator (http://www.stat.ubc.ca/~rollin/stats/ssize/index.html; parameters: power,.80; alpha,.05). When applicable, sample size analysis was based on average values obtained from preliminary optimization/validation trials. When comparing multiple data pools, Kruskal-Wallis test followed by Mann-Whitney U test between two target populations were applied and significance was scored when P<0.05 for both tests. When comparing two data pools, Mann-Whitney U test was applied and significance scored when P<0.05. All statistical analyses were conducted using Prism v5.0 (GraphPad).

### Example 7. Study approval

All protocols and experiments on mice and murine samples were performed in compliance with the Belgian law and the Ethical Approval of KU Leuven.

RNA-seq and miRNA-seq data have been deposited in GEO NCBI under the accession code GSE102283.

### Example 8. Modified expression profile of tumor cells.

The miRNA and miRNA inhibitory cocktail has also been applied on rhabdomyosarcoma cancer cells, which convert the cells into cells expressing Myogenin, thus resembling differentiated myogenic cells. This finding is of potential relevance in tumour treatment whereby tumor cells can be converted in a less detrimental cell type.

### Example 9. Determination of a minimal set of myogenic inducers

The present application discloses sets of compounds that increase the myogenic potential of mesodermal iPSC-derived progenitors of fibroblast origin.

These markers have been initially validated by applying them as a cocktail of compounds to the fibroblast derived cells.

It is however envisaged that the same effect can be achieved with a limited set of compounds.

A cell-based assay is developed wherein individual miRNAs or inhibitors are applied to cells to determine whether single compounds have the same effect as the cocktails described in the above examples. If single compounds are found to be inactive, or partially active, combinations of 2, 3 or more compounds can be tested in an arrayed assay of cells in a 96 or 256 well platform. Activity can be easily screened by e.g. qPCR for one or more markers genes for myogenic potential as described above. Compositions of one or more compounds with promising properties are validated in one of the above described animal models.

## Claims

1. An in vitro method to increase the myogenic potential of progenitor cells from pluripotent stem cells induced into the mesodermal lineage, the method comprising the step of administering to said progenitor cells:
- miR-212, miR -132, miR -424, miR -146b, and miR -181a or mimics thereof, and
- inhibitors for miR-34c-5p, miR-34c-3p, miR-362, miR-210, and miR-590.

2. The method according to claim 1, wherein the pluripotent cells are embryonic stem cells.

3. The method according to claim 1, wherein the pluripotent cells are iPSCs.

4. The method according to claim 3, wherein the iPSCs are from fibroblast lineage.

5. The method according to claim 3 or 4, wherein the iPSCs are from mesoangioblasts.

6. The method according to any one of claims 1 to 5, wherein the mixture further comprises small interfering RNA against one more selected from the group of OSTN, MYB, LHX2, LTBP4, BMP6 and SMAD5.

7. The method according to any one of claims 1 to 6, wherein the mixture comprises:
- small interfering RNA against OSTN, MYB, LHX2, LTBP4, BMP6 and SMAD5, and
- inhibitors for miR-34c-5p, miR-34c-3p, miR-362, miR-210, and miR-590, and
- miR-212, miR -132, miR -424, miR -146b, and miR -181a or mimics thereof.

8. A kit comprising :
miR-212, miR -132, miR -424, miR -146b, and miR -181a or mimics thereof, and,
- inhibitors for miR-34c-5p, miR-34c-3p, miR-362, miR-210, and miR-590.

9. The kit according to any one of claims 8, wherein the mixture further comprises small interfering RNA against one more selected from the group of OSTN, MYB, LHX2, LTBP4, BMP6 and SMAD5.

10. The kit according to claim 8 or 9, wherein the mixture comprises:
- small interfering RNA against OSTN, MYB, LHX2, LTBP4, BMP6 and SMAD5, and
- inhibitors for miR-34c-5p, miR-34c-3p, miR-362, miR-210, and miR-590 and
- miR-212, miR -132, miR -424, miR -146b, and miR -181a or mimics thereof.

11. In vitro use of a kit according to any one of claims 8 to 10 for increasing the myogenic potential of progenitor cells from pluripotent stem cells induced into the mesodermal lineage.

## Patentansprüche

1. Ein in-vitro-Verfahren zur Erhöhung des myogenen Potenzials von Vorläuferzellen aus pluripotenten Stammzellen, die in die mesodermale Linie induziert wurden, wobei das Verfahren den Schritt der Verabreichung von
miR-212, miR-132, miR-424, miR-146b und miR-181a oder deren Mimetika,
sowie von
Inhibitoren für miR-34c-5p, miR-34c-3p, miR-362, miR-210, und miR-590
an die Vorläuferzellen aufweist.

2. Das Verfahren nach Anspruch 1, wobei die pluripotenten Zellen embryonale Stammzellen darstellen.

3. Das Verfahren nach Anspruch 1, wobei die pluripotenten Zellen iPS-Zellen darstellen.

4. Das Verfahren nach Anspruch 3, wobei die iPS-Zellen von der Fibroblasten-Linie stammen.

5. Das Verfahren nach Anspruch 3 oder 4, wobei die iPS-Zellen von Mesoangioblasten stammen.

6. Das Verfahren nach einem der Ansprüche 1 bis 5, wobei das Gemisch, gegenüber einer weiteren, aus der Gruppe OSTN, MYB, LHX 2, LTBP4, BMP6 und SMAD5 ausgewählten RNA, ferner gering interferierende RNA aufweist.

7. Das Verfahren nach einem der Ansprüche 1 bis 6, wobei das Gemisch aufweist:
gegenüber OSTN, MYB, LHX2, LTBP4, BMP6 und SMAD5 gering interferierende RNA,
und
Inhibitoren für miR-34c-5p, miR-34c-3p, miR-362, miR-210, und miR-590,
sowie
miR-212, miR-132, miR-424, miR-146b und miR-181a oder deren Mimetika.

8. Ein Kit, aufweisend:
miR-212, miR-132, miR-424, miR-146b und miR-181a oder deren Mimetika,
und
Inhibitoren für miR-34c-5p, miR-34c-3p, miR-362, miR-210, und miR-590.

9. Der Kit nach Anspruch 8, wobei das Gemisch, gegenüber einer weiteren, aus der Gruppe OSTN, MYB, LHX2, LTBP4, BMP6 und SMAD5 ausgewählten RNA, ferner eine gering interferierende RNA aufweist.

10. Kit nach Anspruch 8 oder 9, wobei das Gemisch aufweist:
eine gegenüber OSTN, MYB, LHX2, LTBP4, BMP6 und SMAD5 gering interferierende RNA
und
Inhibitoren für miR-34c-5p, miR-34c-3p, miR-362, miR-210, und miR-590
sowie
miR-212, miR-132, miR-424, miR-146b und miR-181a oder deren Mimetika.

11. In-vitro-Verwendung eines Kits nach einem der Ansprüche 8 bis 10 zur Erhöhung des myogenen Potenzials von Vorläuferzellen aus pluripotenten Stammzellen, die in die mesodermale Linie induziert wurden.

## Revendications

1. Procédé *in vitro* pour augmenter le potentiel myogène de cellules progénitrices issues de cellules souches pluripotentes induites dans la lignée mésodermique, le procédé comprenant l'étape consistant à administrer auxdites cellules progénitrices :
- miR-212, miR -132, miR -424, miR -146b, et miR - 181a ou leurs mimiques, et
- des inhibiteurs de miR-34c-5p, miR-34c-3p, miR-362, miR-210, et miR-590.

2. Procédé selon la revendication 1, dans lequel les cellules pluripotentes sont des cellules souches embryonnaires.

3. Procédé selon la revendication 1, dans lequel les cellules pluripotentes sont des cellules souches pluripotentes induites CSPi.

4. Procédé selon la revendication 3, dans lequel les CSPi sont de la lignée des fibroblastes.

5. Procédé selon la revendication 3 ou 4, dans lequel les CSPi proviennent de méso-angioblastes.

6. Procédé selon l'une quelconque des revendications 1 à 5, dans lequel le mélange comprend en outre un petit ARN interférent contre un autre élément choisi dans le groupe de OSTN, MYB, LHX2, LTBP4, BMP6 et SMAD5.

7. Procédé selon l'une quelconque des revendications 1 à 6, dans lequel le mélange comprend :
- un petit ARN interférent contre OSTN, MYB, LHX2, LTBP4, BMP6 et SMAD5, et
- des inhibiteurs de miR-34c-5p, miR-34c-3p, miR-362, miR-210, et miR-590, et
- miR-212, miR -132, miR -424, miR -146b, et miR - 181a ou leurs mimiques.

8. Kit comprenant :
- miR-212, miR -132, miR -424, miR -146b, et miR - 181a ou leurs mimiques, et,
- des inhibiteurs de miR-34c-5p, miR-34c-3p, miR-362, miR-210, et miR-590.

9. Kit selon l'une quelconque des revendications 8, dans lequel le mélange comprend en outre un petit ARN interférent contre un autre élément choisi dans le groupe de OSTN, MYB, LHX2, LTBP4, BMP6 et SMAD5.

10. Kit selon la revendication 8 ou 9, dans lequel le mélange comprend :
- un petit ARN interférent contre OSTN, MYB, LHX2, LTBP4, BMP6 et SMAD5, et
- des inhibiteurs de miR-34c-5p, miR-34c-3p, miR-362, miR-210, et miR-590 et
- miR-212, miR -132, miR -424, miR -146b, et miR - 181a ou leurs mimiques.

11. Utilisation *in vitro* d'un kit selon l'une quelconque des revendications 8 à 10 pour augmenter le potentiel myogène de cellules progénitrices issues de cellules souches pluripotentes induites dans la lignée mésodermique.
